# EUROPEAN PATENT APPLICATION

(11) **EP 4 585 244 A1**
(43) Date of publication of application: **16.07.2025**
(21) Application number: 24861443.0
(22) Date of filing: 19.01.2024
(51) Int. Cl.: A61M 16/00, F04D 29/66

(54) **VENTILATOR**

(30) Priority: 10.09.2023 CN 202311167360
(71) Applicant: Jiangsu Yuwell Medical Equipment & Supply Co., Ltd., Zhenjiang, Jiangsu 212300 (CN); Nanjing Yuwell Software Technology Co., Ltd., Nanjing, Jiangsu 210023 (CN); Suzhou Yuwell Medical Technology Co., Ltd., Suzhou, Jiangsu 215600 (CN)
(72) Inventor: LIU, Yun, Zhenjiang, Jiangsu 212300 (CN); GUO, Jianming, Zhenjiang, Jiangsu 212300 (CN); ZHU, Tingting, Zhenjiang, Jiangsu 212300 (CN); ZHAO, Shuai, Zhenjiang, Jiangsu 212300 (CN); WU, Qun, Zhenjiang, Jiangsu 212300 (CN)
(74) Representative: Huang, Liwei
(86) International application number: PCT/CN2024/073254
(87) International publication number: WO 2025/050592

(57) **Abstract**

The present invention discloses a respirator. A housing has an air inlet communicating with outside atmosphere; a separator plate is arranged on the housing and provided with an air blower containing cavity, and an air blower body is mounted in the air blower containing cavity; an upper cover plate is arranged on the separator plate; and the respirator is configured that a first cavity is formed between the separator plate and the housing, a space between the separator plate and the upper cover plate is divided by the air blower body into a second cavity located in front of an air entering port of the air blower body and a flow monitoring cavity located behind an air outlet port of the air blower body, and an air flow channel communicating with the first cavity and the second cavity is formed in the separator plate. By using this solution, a whole structure of the respirator is more compact and reliable, the number of components of the whole respirator is reduced, the structure is ingenious and reasonable, fitting is simple, fitting efficiency and fitting quality are improved, thus safety and reliability of using the respirator by a patient are improved, and the respirator, as a medical supply, may make the patient use it more safely and more stably.

## Description

The present invention claims priority to Chinese Patent Application No. 202311167360.6 filed with CNIPA on September 10, 2023 and entitled "RESPIRATOR", the entire contents of which are incorporated herein by reference.

### TECHNICAL FIELD

The present invention relates to the technical field of medical equipment, in particular to a respirator.

### BACKGROUND

A respirator, as an effective means for artificially replacing an autonomous ventilation function, has been widely applied to respiratory failure caused by different reasons, anesthesia respiratory management during major surgery, breath support treatment and resuscitation et premiers secours, holds the important position in the field of modern medicine and is a vital medical device capable of playing a role in preventing and treating respiratory failure, reducing complication and saving and prolonging patients' lives.

At present, a respirator on the market is mainly composed of two parts, namely, a chasis with air path and a humidifier. During working of the respirator, a signal of respiratory obstruction of a patient is detected through a controller, air in an air circuit of the respirator is pressurized appropriately, air is blown into the humidifier through an air blower in the host device, the air is humidified through the humidifier to be fed into a respiratory tract of a user, then the obstructed respiratory tract of the patient is forced open, continuous ventilation is provided for the patient, and the patient can keep inhaling enough oxygen.

During implementing the present invention, the applicant discovers that an existing respirator at least has the following defects.
1. An air path module part and a host device housing part are merely in a fitting relationship, many spare holes inside the housing are not utilized, the original air path module part occupies a larger space, the internal structure of the respirator is loose, which increase the whole size of the respirator, and cause hidden risks such as reducing the use effect exist during use.
2. An extra air path needs to be arranged for air coming from the outside of the respirator, an extra air path or pipe also needs to be arranged for implementing a function of detecting the flow quantity, the number of components is large, fitting difficulty is increased, and fitting efficiency and fitting quality are affected.
3. A corresponding designed air flow runner depends on various optimal designs of the air path module part, which makes the purposes of stable flow and low noise are difficult to achieve.

In view of this, how to work out and design a respirator to solve problems in the prior art is an issue to be solved and researched by the present invention.

### SUMMARY

An objective of the present invention is to provide a respirator.

In order to achieve the above-mentioned objective, the present invention provides a respirator. The respirator comprises:
a housing, provided with an air inlet communicating with outside atmosphere;
a separator plate, arranged on the housing and provided with an air blower containing cavity, wherein an air blower body is mounted in the air blower containing cavity; and
an upper cover plate, arranged on the separator plate;
wherein the respirator is configured that a first cavity is formed between the separator plate and the housing, a space between the separator plate and the upper cover plate is divided by the air blower body into a second cavity located in front of an air entering port of the air blower body and a flow monitoring cavity located behind an air outlet port of the air blower body, and an air flow channel communicating with the first cavity and the second cavity is formed in the separator plate.

The related contents of the present invention are explained as follows.
1. In the above-mentioned embodiment of the present invention, the respirator is mainly designed as a structure with the housing, the separator plate and the upper cover plate, the first cavity is formed between the separator plate and the housing, the position between the separator plate and the upper cover plate is divided by the air blower body into the second cavity located in front of the air entering port of the air blower body and the flow monitoring cavity located behind the air outlet port of the air blower body, the air flow channel communicating with the first cavity and the second cavity is formed in the separator plate, and in this way, an air path module originally separately mounted in the housing of the respirator is optimized and simplified, so the housing of the respirator can be a portion of the air path module; the first cavity is formed between the housing and the separator plate, air entering the air path module of the respirator enters the first cavity from the air inlet, then enters the second cavity formed by the separator plate and the upper cover plate from the air flow channel, is blown into the flow monitoring cavity through the air blower body and then enters a humidifier; by means of this flow channel design, the first cavity formed between the housing and the separator plate is used as an air intaking path of an air entering flow path, and an air flow may be stabilized and air flow noise may be reduced without additionally arranging corresponding pipes; and spare gaps in an original housing may be fully utilized, meanwhile, by using the second cavity and the flow monitoring cavity formed between the separator plate and the upper cover plate, the function of monitoring the air flow may be implemented by this arrangement without arrangement of extra pipes, a whole structure of the respirator is more compact and reliable, the number of components of the whole respirator is reduced, the structure is ingenious and reasonable, fitting is simple, fitting efficiency and fitting quality are improved, thus the safety and reliability of using the respirator by a patient are improved, and the respirator, as a medical supply, may make the patient use it more safely and more stably.
2. In the above-mentioned embodiment, a flexible sealing member is integrally formed on partical surface and an outer edge of the separator plate through a mold, the flexible sealing member is configured to at least act on a connection position between the separator plate and the housing and a connection position between the separator plate and the upper cover plate, and the flexible sealing member formed with the separator plate seals all fitting components of the first cavity, the second cavity and the flow monitoring cavity, so that the number of extra components may be reduced, and fitting efficiency and quality are improved.
3. In the above-mentioned embodiment, a plurality of ribs are extended out of the flexible sealing member and are arranged in the air blower containing cavity of the separator plate in a surrounding manner, and the plurality of ribs constitute an elastic support for mounting the air blower body to elastically limit the air blower body; the elastic support is extended out of the flexible sealing member, on the one hand, extra components do not need to be added, and on the other hand, the air blower body may be elastically limited by using self-elasticity and friction force of the flexible component, so that good flexible connection and buffering can be achieved between the air blower body and the separator plate, the design is ingenious, vibration of the air blower body can be reduced effectively, meanwhile, sufficient friction force and limiting can also guarantee firm mounting of the air blower body, and vibration and noise reduction are achieved; and the flow monitoring cavity is also formed by joint connection and cooperation of the flexible sealing member, an air outlet port joint of the air blower body, the separator plate and the upper cover plate, and the air outlet port joint of the air blower body also acts and cooperates with the flexible sealing member, which also plays a role in vibration and noise reduction.
4. In the above-mentioned embodiment, a first side plate for separating the first cavity is arranged at a lower side edge of the separator plate, a second side plate, corresponding to the first side plate, is arranged on the housing, a sealing strip is extended out of the flexible sealing member and is arranged on an outer side of the first side plate, the separator plate is arranged in the housing and then separates the first cavity by airtight cooperation of the first side plate, the sealing strip and the second side plate; the first side plate and the second side plate are configured to face inward and tilt downward, so that separation for the first cavity is implemented by using self-structures of the separator plate and the housing; and integrated sealing is implemented by using the sealing strip extended out of the flexible sealing member, and extra sealing components do not need to be arranged, so fitting steps are reduced, labor time is saved, and a sealing effect is also improved.
5. In the above-mentioned embodiment, an annular sealing ring is further extended out of the sealing strip of the flexible sealing member and located on a round bottom surface of the separator plate, the round bottom surface of the separator plate is closely connected with the housing through the sealing ring, so that an annular air intaking path is formed in the first cavity, air entering the first cavity can flow around the annular air intaking path and then enter the second cavity through the air flow channel, and the annular air intaking path will make the air flow smoother, and has a certain effect on lowering noise; and meanwhile, bottom sealing is also introduced from the flexible sealing member integrated with the separator plate, assembling is convenient, and the sealing effect is good.
6. In the above-mentioned embodiment, the upper cover plate is provided with a first sampling port communicating with the first cavity, a second sampling port communicating with the second cavity and a third sampling port communicating with the flow monitoring cavity, and monitoring sensors, corresponding to the first sampling port, the second sampling port and the third sampling port, are arranged on a circuit board of the respirator. All the sampling ports are arranged in this way, so the air flow in this position is stable, measurement is accurate, meanwhile, the sampling ports are integrally jointed to the upper cover plate, the sampling ports directly communicate with the first cavity, the second cavity and the flow monitoring cavity, all the monitoring sensors are also connected with the corresponding sampling ports, to monitor air flow parameters such as a high pressure, a low pressure and a flow quantity, arrangement of an extra monitoring air path is avoided, and the number of components such as joints and pipes is reduced.
7. In the above-mentioned embodiment, the upper cover plate is provided with a sunk area, the first sampling port, the second sampling port and the third sampling port are formed in the sunk area, so that a structure of an air path monitoring part of the respirator is more compact, and a whole size of the respirator can be controlled effectively.
8. In the above-mentioned embodiment, the circuit board is provided with an adapter sealing member corresponding to the sunk area where the first sampling port, the second sampling port and the third sampling port are located, the first sampling port, the second sampling port, the third sampling port and the corresponding monitoring sensors are integrally sealed and adapter-connected through one adapter sealing member, the number of components is small, and fitting is convenient.
9. In the above-mentioned embodiment, the first sampling port, the second sampling port and the third sampling port are joint pipe structures formed integrally with the upper cover plate, the separator plate is provided with a sampling extending pipe configured to communicate with the first cavity and the first sampling port, so that air runners of the corresponding sampling ports are formed, and abutting joint of the sensors and sealing and adapter-connecting of the adapter sealing member may be more conveniently monitored by means of the arranged joint pipe structures.
10. In the above-mentioned embodiment, the upper cover plate is provided with a plurality of sunk areas, another sunk areas are configured to contain an electronic element distributed downwards on a lower surface of the circuit board, another sunk areas here refer to sunk areas except the sunk area where the first sampling port, the second sampling port and the third sampling port are located, so that the electronic elements protruding on the circuit board can be contained downwards in the sunk area of the upper cover plate, and a vertical height of the respirator is reduced.
11. In the present invention, a steering pipe is arranged at the air flow channel of the separator plate, the steering pipe is provided with a first steering port located in the second cavity and a second steering port located in the first cavity, and a laminar flow element is arranged in the steering pipe; and a whole structure of the steering pipe is located on the separator plate and the upper cover plate and between the second cavity and the first cavity, air entering the first cavity from the air inlet enters a first end of the steering pipe along the air intaking path, then enters the second cavity from a second end of the steering pipe and enters the air blower body via a blowing runner, the steering pipe is not arranged outside or on the periphery, so an external space of the air path module is not occupied, which also achieves space optimization, and the steering pipe is integrated with the separator plate, so the number of components is reduced.

By using the above solution, compared with the prior art, the present invention has the following advantages and effects.
1. In the above embodiment of the present invention, the respirator is mainly designed as a structure with the housing, the separator plate and the upper cover plate, the first cavity is formed between the separator plate and the housing, the position between the separator plate and the upper cover plate is divided by the air blower body into the second cavity located in front of the air entering port of the air blower body and the flow monitoring cavity located behind the air outlet port of the air blower body, and the air flow channel communicating with the first cavity and the second cavity is formed in the separator plate, so that the air path module originally separately mounted in the housing of the respirator is optimized and simplified, and the housing of the respirator can also be a portion of the air path module.
2. In the above embodiment of the present invention, the first cavity is formed between the housing and the separator plate, air entering the air path module of the respirator enters the first cavity from the air inlet, then enters the second cavity formed by the separator plate and the upper cover plate from the air flow channel, is blown into the flow monitoring cavity by the air blower body and then enters the humidifier, by using this runner design, the first cavity formed between the housing and the separator plate is used as the air intaking path of the air entering flow path, the air flow may be stabilized and air flow noise may be reduced without arrangement of extra corresponding pipes, and spare gaps in the original housing may be fully utilized.
3. In the above embodiment of the present invention, by using the second cavity and the flow monitoring cavity formed between the separator plate and the upper cover plate, the function of monitoring the air flow may be implemented by this arrangement without arrangement of extra pipes, the number of components is reduced, and the size of the respirator is reduced.
4. To sum up, the embodiment of the present invention may stabilize the air flow and reduce the air flow noise by ingeniously using the first cavity formed between the housing and the separator plate as the air intaking path of the air entering flow path without arrangement of extra corresponding pipes, fully utilize the spare gaps in the original housing and form the second cavity and the flow monitoring cavity between the separator plate and the upper cover plate, so that a whole structure of the respirator is more compact and reliable, the number of components of the whole respirator is reduced, the structure is ingenious and reasonable, fitting is simple, fitting efficiency and fitting quality are improved, thus the safety and reliability of using the respirator by a patient are improved, and the respirator, as a medical supply, may make the patient use it more safely and more stably.

### BRIEF DESCRIPTION OF THE DRAWINGS

Fig. 1 is a schematic structural diagram of a respirator in an embodiment of the present invention.
Fig. 2 is a schematic diagram of fitting of a respirator in an embodiment of the present invention.
Fig. 3 is a schematic explosive view of a respirator in an embodiment of the present invention.
Fig. 4 is a schematic diagram of fitting of components such as a separator plate, an upper cover plate and an air blower body in an embodiment of the present invention.
Fig. 5 is a schematic diagram of fitting an air blower body and a separator plate in a housing in an embodiment of the present invention.
Fig. 6 is a schematic top view of a respirator of which a part of structure is removed in an embodiment of the present invention.
Fig. 7 is a schematic diagram of a housing in an embodiment of the present invention.
Fig. 8 is a schematic three-dimensional diagram of a separator plate in an embodiment of the present invention (first viewing angle).
Fig. 9 is a schematic three-dimensional diagram of a separator plate in an embodiment of the present invention (second viewing angle).
Fig. 10 is a schematic three-dimensional diagram of an upper cover plate in an embodiment of the present invention.
Fig. 11 is a schematic sectional view of an upper cover plate in an embodiment of the present invention (first direction).
Fig. 12 is a schematic sectional view of an upper cover plate in an embodiment of the present invention (second direction).
Fig. 13 is a schematic three-dimensional diagram of an adapter sealing member in an embodiment of the present invention (first viewing angle).
Fig. 14 is a schematic three-dimensional diagram of an adapter sealing member in an embodiment of the present invention (second viewing angle).
Fig. 15 is a schematic sectional view of an adapter sealing member in an embodiment of the present invention (first direction).
Fig. 16 is a schematic sectional view of an adapter sealing member in an embodiment of the present invention (second direction).
Fig. 17 is a schematic sectional view of a respirator in an embodiment of the present invention (first direction).
Fig. 18 is a schematic sectional view of a respirator in an embodiment of the present invention (second direction).
Fig. 19 is a schematic sectional view of a respirator in an embodiment of the present invention (third direction).
Fig. 20 is a schematic sectional view of a respirator in an embodiment of the present invention (fourth direction).
Fig. 21 is a schematic diagram of a whole air flow direction of a respirator in an embodiment of the present invention.
Fig. 22 is a schematic diagram of a flow direction in which an air flow enters a first cavity from outside in a respirator in an embodiment of the present invention.
Fig. 23 is a schematic diagram of a flow direction in which an air flow enters a second cavity from a first cavity in a respirator in an embodiment of the present invention.
Fig. 24 is a schematic diagram of a flow direction in which an air flow enters an air blower body from a second cavity in a respirator in an embodiment of the present invention.
Fig. 25 is a schematic diagram of a flow direction in which an air flow enters a flow monitoring cavity from a second cavity in a respirator in an embodiment of the present invention.

### EXPLANATION OF REFERENCE NUMERALS

1, housing; 10, containing space; 11, air inlet; 111, air entering pipe; 12, second side plate; 2, separator plate; 21, air blower containing cavity; 22, air flow channel; 220, steering pipe; 221, first steering port; 222, second steering port; 223, laminar flow element; 23, flexible sealing member; 231, rib; 232, sealing strip; 233, sealing ring; 24, first side plate; 25, sampling extending pipe; 3, air blower body; 31, air entering port; 32, air outlet port; 4, upper cover plate; 41, first sampling port; 42, second sampling port; 43, third sampling port; 44, sunk area; 5, circuit board; 51, monitoring sensor; 52, adapter sealing member; 521, first adapter portion; 522, second adapter portion; 523, third adapter portion; 53, electronic element; 60, first cavity; 70, second cavity; 80, flow monitoring cavity; and 9, humidifier.

### DETAILED DESCRIPTION OF THE EMBODIMENTS

In order to make the above objectives, features and advantages of the present invention clearer and easier to understand, specific embodiments of the present invention are described in detail below with reference to the accompanying drawings. Many details are described in the following description for fully understanding the present invention. However, the present invention can be implemented in many other manners different from those described here. Those skilled in the art can make similar improvements without violating an intention of the present invention, so the present invention is not limited by specific embodiments disclosed below.

In the present invention, unless otherwise clearly specified and limited, terms such as "mount", "connected", "connection" and "fixed" are to be understood in a broad sense, for example, it may be a fixed connection or a detachable connection, or may be integrated; it may be a mechanical connection, direct connection, or an indirect connection through an intermediate medium, and two elements may internally communicate, or it may be an interaction relationship between the two elements, unless otherwise clearly limited. Specific meanings of the above terms in the present invention may be understood by those ordinarily skilled in the art according to specific conditions.

In the present invention, a direction or position relationship indicated by terms such as "upper", "lower", "bottom", "inner" and "outer" is a direction or position fitting relationship indicated based on the accompanying drawings, which is merely intended to conveniently describe the present invention and simplify the description but not to indicate or imply that the referred to apparatus or element necessarily has the specific direction, or constructed and operated in the specific direction, and thus, it cannot be understood as a limitation on the present invention.

Besides, a term "and/or" in the present invention means that there are three parallel solutions, and taking "A and/or B" as an example, it includes a solution of only A, a solution of only B, or a solution of both A and B.

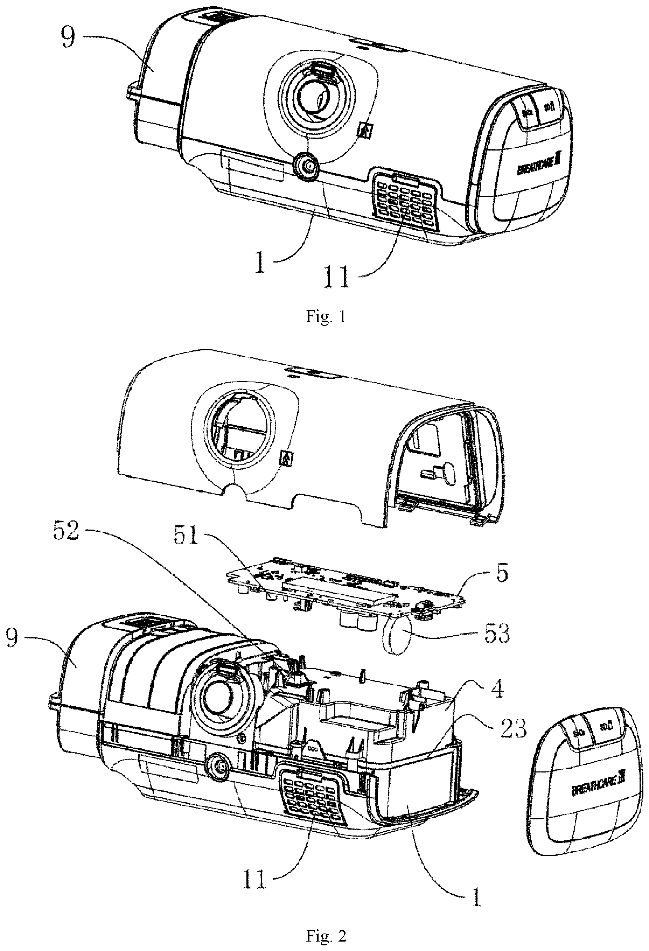

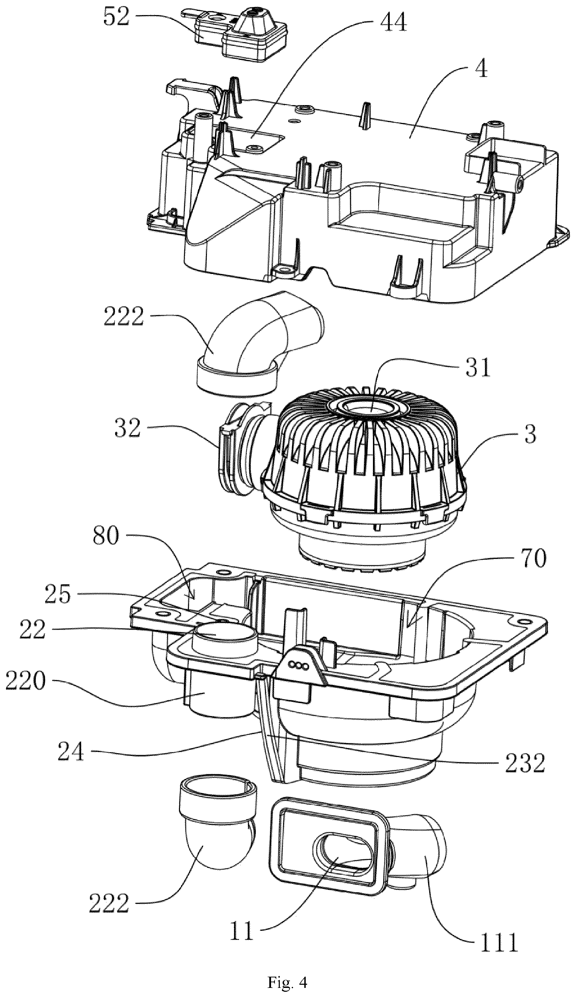

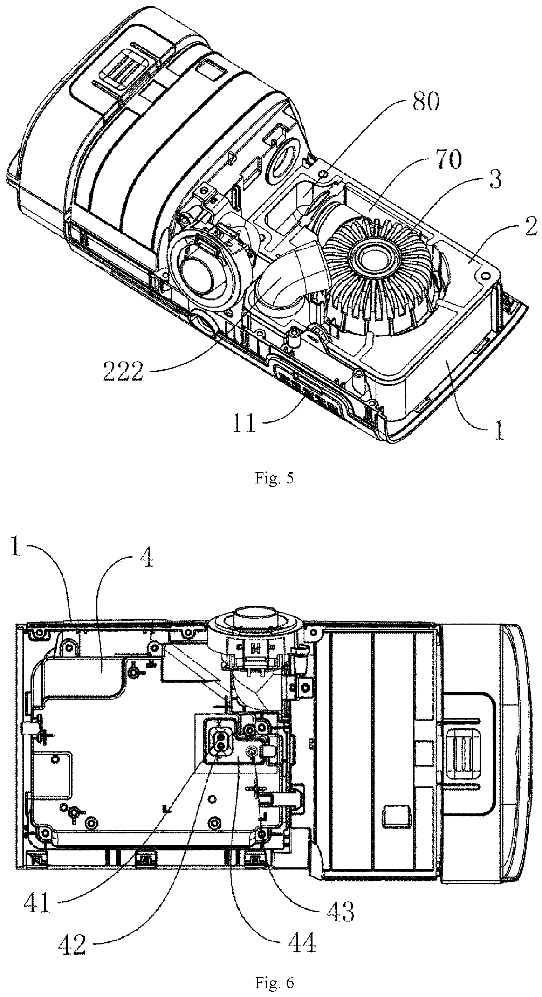

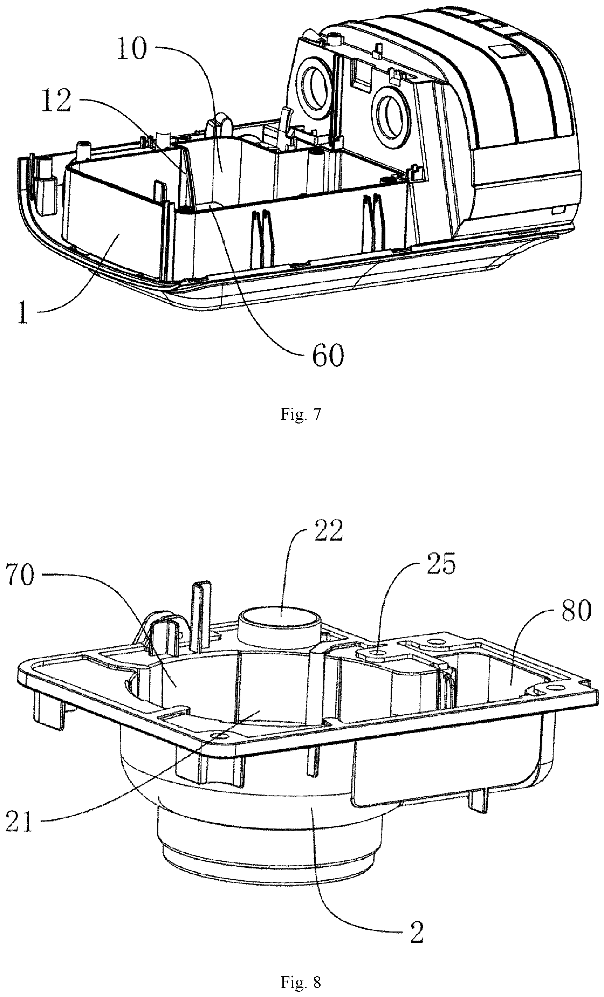

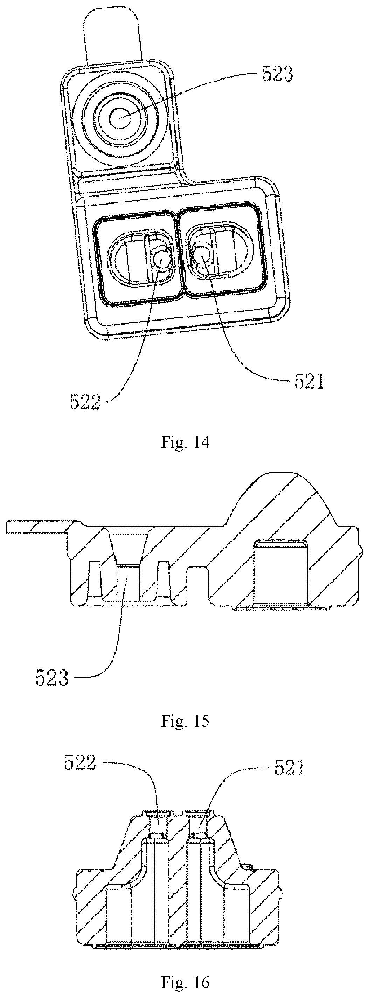

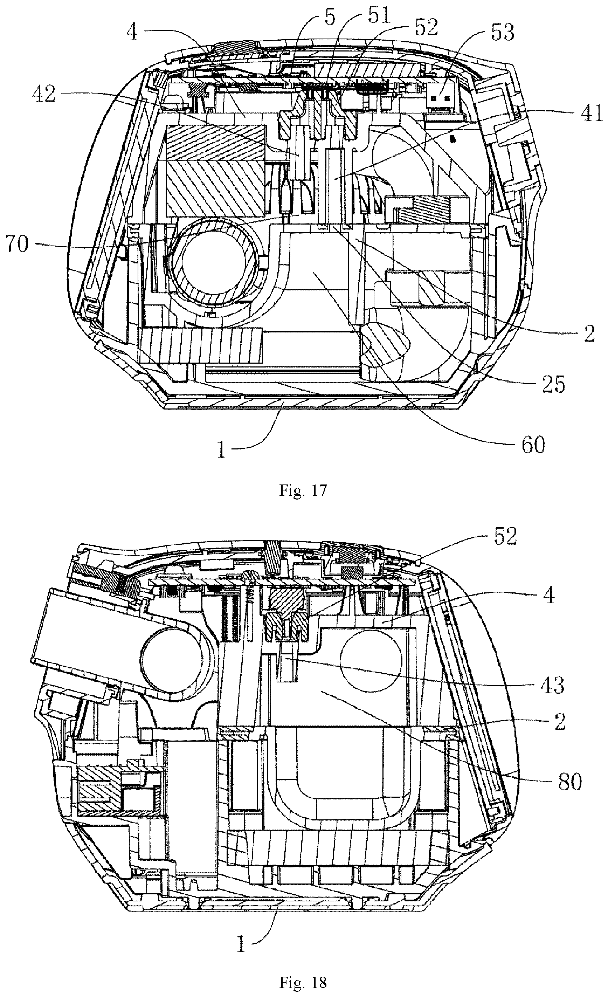

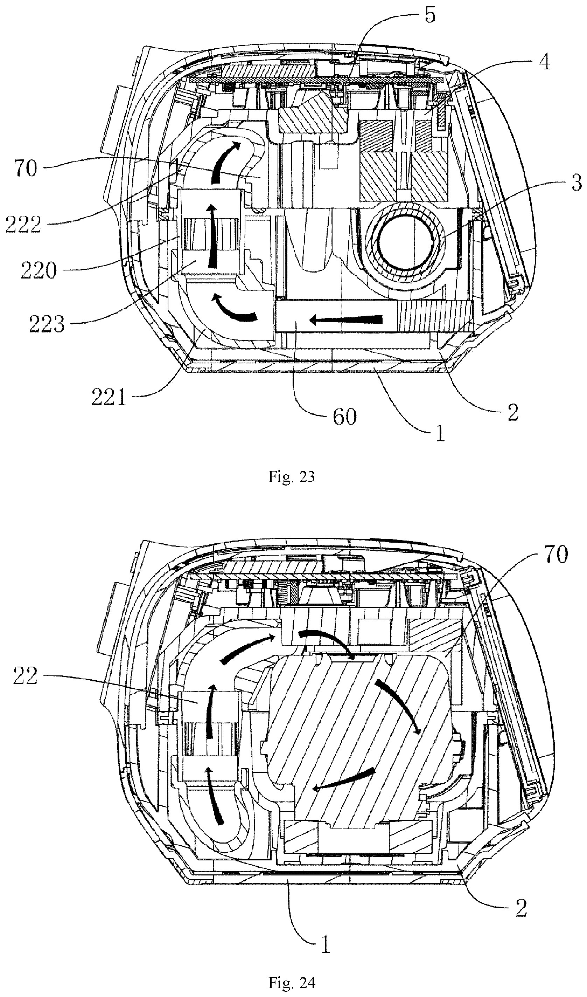

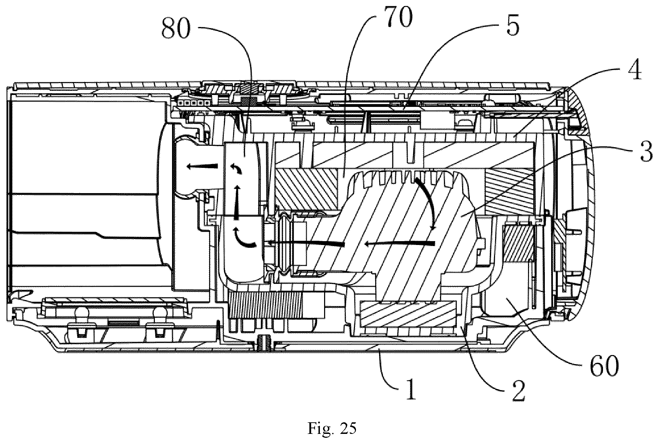

As shown in Fig. 1 to Fig. 25, an embodiment of the present invention discloses a respirator which is provided with:
a housing 1, wherein the housing 1 has an air inlet 11 communicating with the outside atmosphere; a separator plate 2, wherein the separator plate 2 is arranged on the housing 1 and provided with an air blower containing cavity 21, and an air blower body 3 is mounted in the air blower containing cavity 21; and an upper cover plate 4, wherein the upper cover plate 4 is arranged on the separator plate 2.

In the embodiment of the present invention, the respirator is configured that a first cavity 60 is formed between the separator plate 2 and the housing 1, a space between the separator plate 2 and the upper cover plate 4 is divided by the air blower body 3 into a second cavity 70 located in front of an air entering port 31 of the air blower body 3 and a flow monitoring cavity 80 located behind an air outlet port 32 of the air blower body 3, and an air flow channel 22 communicating with the first cavity 60 and the second cavity 70 is formed in the separator plate 2.

In the embodiment of the present invention, the respirator is mainly designed as a structure with the housing 1, the separator plate 2 and the upper cover plate 4, the first cavity 60 is formed between the separator plate 2 and the housing 1, the position between the separator plate 2 and the upper cover plate 4 is divided by the air blower body 3 into the second cavity 70 located in front of the air entering port 31 of the air blower body 3 and the flow monitoring cavity 80 located behind the air outlet port 32 of the air blower body 3, and the air flow channel 22 communicating with the first cavity 60 and the second cavity 70 is formed in the separator plate 2, so that an air path module originally separately mounted in the housing 1 of the respirator is optimized and simplified, and the housing 1 of the respirator can also be a portion of the air path module; and by using the second cavity 70 and the flow monitoring cavity 80 formed between the separator plate 2 and the upper cover plate 4, the function of monitoring the air flow may be implemented by this arrangement without arrangement of extra pipes, and a whole structure of the respirator is more compact and more reliable.

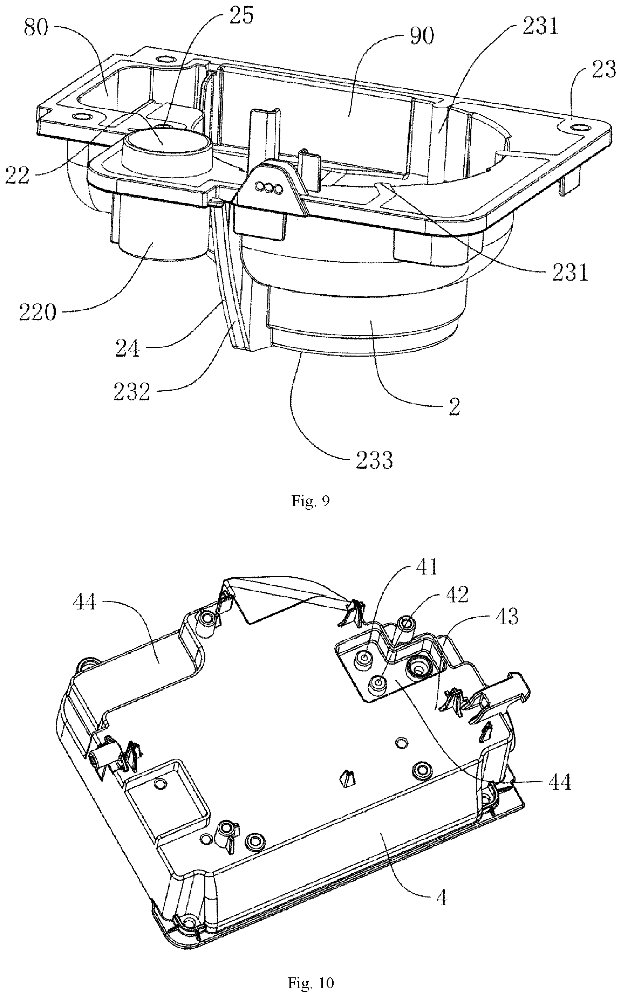

In the embodiment of the present invention, referring to Fig. 9, a flexible sealing member 23 is integrally formed on partial surface and an outer edge of the separator plate 2 through a mold, the flexible sealing member 23 is configured to at least act on a connection position between the separator plate 2 and the housing 1 and a connection position between the separator plate 2 and the upper cover plate 4, and the flexible sealing member 23 formed with the separator plate 2 seals all fitting components of the first cavity 60, the second cavity 70 and the flow monitoring cavity 80, so that the number of extra components may be effectively reduced, and fitting efficiency and quality are improved.

In the embodiment of the present invention, referring to Fig. 9, a plurality of ribs 231 are extended out of the flexible sealing member 23 and are arranged in the air blower containing cavity 21 of the separator plate 2 in a surrounding manner, and the plurality of ribs 231 constitute an elastic support for mounting the air blower body 3 to elastically limit the air blower body 3; the elastic support is extended out of the flexible sealing member 23, on the one hand, extra components do not need to be added, and on the other hand, the air blower body 3 can be elastically limited by using self-elasticity and friction force of the flexible component , so that good flexible connection and buffering can be achieved between the air blower body 3 and the separator plate 2, the design is ingenious, vibration of the air blower body 3 can be reduced effectively, meanwhile, sufficient friction force and limiting can also guarantee firm mounting of the air blower body 3, and vibration and noise reduction are achieved; and the flow monitoring cavity 80 is also formed by joint connection and cooperation of the flexible sealing member 23, an air outlet port 32 joint of the air blower body 3, the separator plate 2 and the upper cover plate 4, and the air outlet port 32 joint of the air blower body 3 also acts and cooperates with the flexible sealing member 23, which also plays a role in vibration and noise reduction.

In the embodiment of the present invention, referring to Fig. 9, a first side plate 24 for separating the first cavity 60 is arranged at a lower side edge of the separator plate 2, a second side plate 12, corresponding to the first side plate 24, is arranged on the housing 1, a sealing strip 232 is extended out of the flexible sealing member 23 and is arranged on an outer side of the first side plate 24, the separator plate 2 is arranged in the housing 1 and then separates the first cavity 60 by airtight cooperation of the first side plate 24, the sealing strip 232 and the second side plate 12; the first side plate 24 and the second side plate 12 are configured to face inward and tilt downward, so that separation for the first cavity 60 is implemented by using self-structures of the separator plate 2 and the housing 1; and integrated sealing is implemented by using the sealing strip 232 extended out of the flexible sealing member 23, and extra sealing components do not need to be arranged, so fitting steps are reduced, labor time is saved, and meanwhile, a sealing effect is also improved.

In the embodiment of the present invention, referring to Fig. 9, an annular sealing ring 233 is further extended out of the sealing strip 232 of the flexible sealing member 23 and located on a round bottom surface of the separator plate 2, the round bottom surface of the separator plate 2 is closely connected with the housing 1 through the sealing ring 233, so that an annular air intaking path is formed in the first cavity 60, air entering the first cavity 60 can flow around the annular air intaking path and then enter the second cavity 70 through the air flow channel 22, and the annular air intaking path will make the air flow smoother, and has a certain effect on lowering noise; and meanwhile, bottom sealing is also introduced from the flexible sealing member 23 integrated with the separator plate 2, assembling is convenient, and the sealing effect is good.

In the embodiment of the present invention, referring to Fig. 10 to Fig. 12, the upper cover plate 4 is provided with a first sampling port 41 communicating with the first cavity 60, a second sampling port 42 communicating with the second cavity 70 and a third sampling port 43 communicating with the flow monitoring cavity 80, and monitoring sensors 51, corresponding to the first sampling port 41, the second sampling port 42 and the third sampling port 43, are arranged on a circuit board 5 of the respirator. All the sampling ports are arranged in this way, so the air flow in this position is stable, measurement is accurate, meanwhile, the sampling ports are integrally jointed to the upper cover plate 4, the sampling ports directly communicate with the first cavity 60, the second cavity 70 and the flow monitoring cavity 80, all the monitoring sensors 51 are connected with the corresponding sampling ports, to monitor air flow parameters such as a high pressure, a low pressure and a flow quantity, arrangement of an extra monitoring air path is avoided, and the number of components such as joints and pipes is reduced. Specifically, the upper cover plate 4 is provided with a sunk area 44, the first sampling port 41, the second sampling port 42 and the third sampling port 43 are formed in the sunk area 44, so that a structure of an air path monitoring part of the respirator is more compact, and a whole size of the respirator can be controlled effectively.

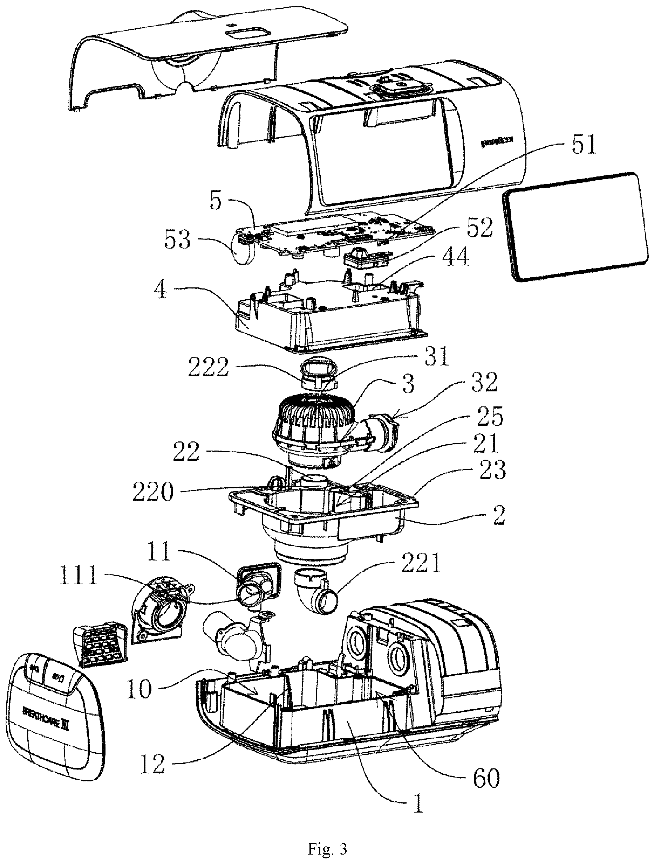

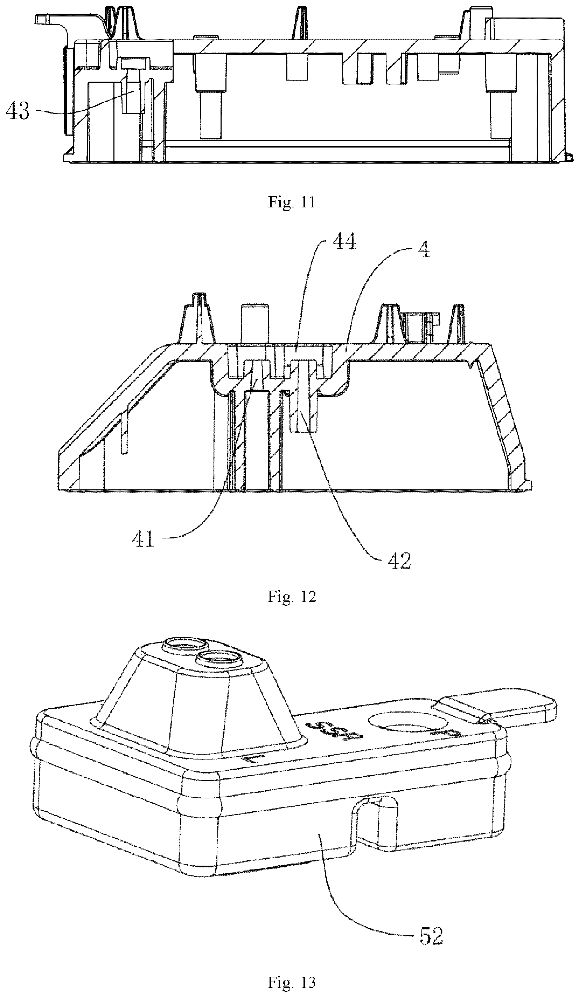

In the embodiment of the present invention, referring to Fig. 3, the circuit board 5 is provided with an adapter sealing member 52 corresponding to the sunk area 44 where the first sampling port 41, the second sampling port 42 and the third sampling port 43 are located, the first sampling port 41, the second sampling port 42, the third sampling port 43 and the corresponding monitoring sensors 51 are integrally sealed and adapter-connected through one adapter sealing member 52, the number of components is small, and fitting is convenient. A structure of the adapter sealing member 52 refers to Fig. 13 to Fig. 16. The adapter sealing member 52 is provided with a first adapter portion 521, a second adapter portion 522 and a third adapter portion 523 corresponding to the first sampling port 41, the second sampling port 42 and the third sampling port 43. Specifically, referring to Fig. 10 to Fig. 12, the first sampling port 41, the second sampling port 42 and the third sampling port 43 are joint pipe structures formed integrally with the upper cover plate 4, the separator plate 2 is provided with a sampling extending pipe 25 configured to communicate with the first cavity 60 and the first sampling port 41, so that the air runners of the corresponding sampling ports are formed, and abutting joint of the sensors 51 and sealing and adapter-connecting of the adapter sealing member 52 may be more conveniently monitored by means of the arranged joint pipe structures.

In the embodiment of the present invention, referring to Fig. 10, the upper cover plate 4 is provided with a plurality of sunk areas 44, another sunk areas 44 are configured to contain an electronic element 53 arranged downwards on a lower surface of the circuit board 5, another sunk areas here refer to sunk areas 44 except the sunk area 44 where the first sampling port 41, the second sampling port 42 and the third sampling port 43 are located, so that the electronic elements 53 protruding on the circuit board 5 can be contained downwards in the sunk area 44 of the upper cover plate 4, and a vertical height of the respirator is reduced.

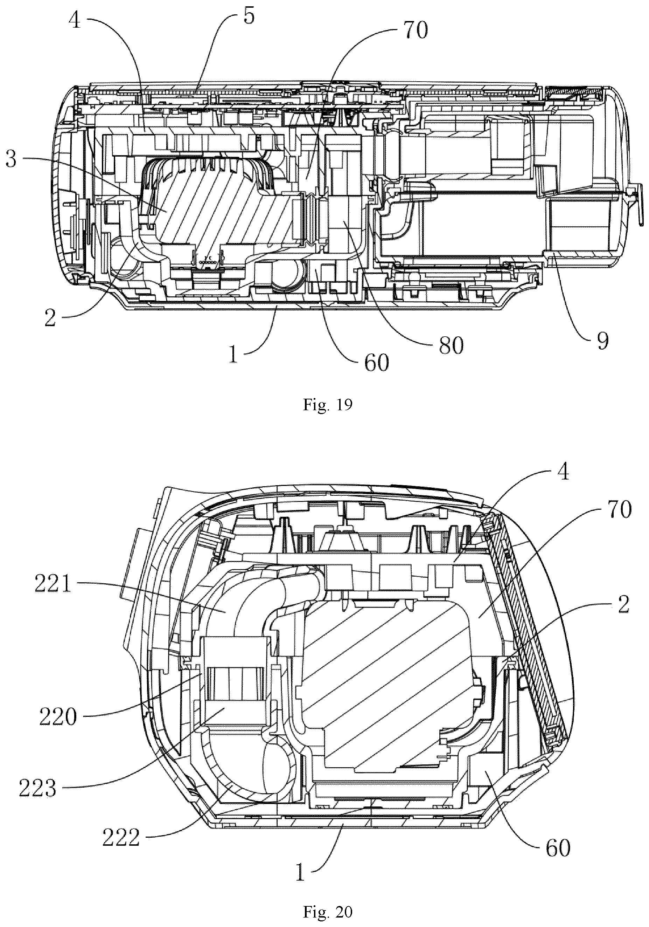

In the embodiment of the present invention, referring to Fig. 20, a steering pipe 220 is arranged at the air flow channel 22 of the separator plate 2, the steering pipe 220 is provided with a first steering port 221 located in the second cavity 70 and a second steering port 222 located in the first cavity 60, and a laminar flow element 223 is arranged in the steering pipe 220; by using the laminar flow element 223 as a boundary, a portion located above the laminar flow element 223 is the second cavity 70, and a portion located below the laminar flow element 223 is the first cavity 60; and a whole structure of the steering pipe 220 is located on the separator plate 2 and the upper cover plate 4 and between the second cavity 70 and the first cavity 60, air entering the first cavity 60 from the air inlet 11 enters a first end of the steering pipe 220 along the air intaking path, then enters the second cavity 70 from a second end of the steering pipe 220 and enters the air blower body 3 via a blowing runner, the steering pipe 220 is not arranged outside or on the periphery, so an external space of the air path module is not occupied, which also achieves space optimization, and the steering pipe 220 is integrated with the separator plate 2, so the number of components is reduced.

In addition, the solution of the present invention is further described in detail by taking one embodiment.

In this embodiment, the respirator is provided with the housing 1, the separator plate 2, the air blower body 3, the upper cover plate 4 and the circuit board 5 as well as the flexible sealing member 23 configured to fit and seal the housing 1, the separator plate 2, the air blower body 3 and the upper cover plate 4, the separator plate 2 and the flexible sealing member 23 are integrally formed through a mold, and the adapter sealing member 52 configured to seal and adapter-connect the circuit board 5 and the sunk area 44 of the upper cover plate 4 is further included.

In this embodiment, the first cavity 60 is formed between the separator plate 2 and the housing 1, the position between the separator plate 2 and the upper cover plate 4 is divided by the air blower body 3 into the second cavity 70 located in front of the air entering port 31 of the air blower body 3 and the flow monitoring cavity 80 located behind the air outlet port 32 of the air blower body 3 , and the air flow channel 22 communicating with the first cavity 60 and the second cavity 70 is formed in the separator plate 2.

In this embodiment, the separator plate 2 is arranged in a containing space 10 of the housing 1, the first side plate 24 for separating the first cavity 60 is arranged at a lower side edge of the separator plate 2, the second side plate 12, corresponding to the first side plate 24, is arranged on the housing 1, the sealing strip 232 is extended out of the flexible sealing member 23 and arranged on the outer side of the first side plate 24, the separator plate 2 is arranged in the housing 1 and then separates the first cavity 60 by airtight cooperation of the first side plate 24, the sealing strip 232 and the second side plate 12; and the first side plate 24 and the second side plate 12 are arranged configured to face inward and tilt downward. The annular sealing ring 233is further extended out of the sealing strip 232 of the flexible sealing member 23 and located on the round bottom surface of the separator plate 2, the round bottom surface of the separator plate 2 is closely connected with the housing 1 through the sealing ring 233, so that an annular air intaking path is formed in the first cavity 60. The steering pipe 220 is arranged at the air flow channel 22 of the separator plate 2, the steering pipe 220 is provided with the first steering port 221 located in the second cavity 70 and the second steering port 222 located in the first cavity 60, and the laminar flow element 223 is arranged in the steering pipe 220.

In this embodiment, the air blower body 3 is mounted in the air blower containing cavity 21 of the separator plate 2, the plurality of ribs 231 are extended out of the flexible sealing member 23 and arranged in the air blower containing cavity 21 of the separator plate 2 in a surrounding manner, and the plurality of ribs 231 constitute the elastic support for mounting the air blower body 3 to elastically limit the air blower body 3.

In this embodiment, the upper cover plate 4 is arranged on the separator plate 2, and the position between the separator plate 2 and the upper cover plate 4 is divided by the air blower body 3 into the second cavity 70 located in front of the air entering port 31 of the air blower body 3 and the flow monitoring cavity 80 located behind the air outlet port 32 of the air blower body 3; and the first sampling port 41 communicating with the first cavity 60, the second sampling port 42 communicating with the second cavity 70 and the third sampling port 43 communicating with the flow monitoring cavity 80 are formed in one sunk area 44 of the upper cover plate 4, and the sunk area 44 is used as an air flow monitoring region. Specifically, the first sampling port 41, the second sampling port 42 and the third sampling port 43 are the joint pipe structures formed integrally with the upper cover plate 4, and the sampling extending pipe 25 configured to communicate with the first cavity 60 and the first sampling port 41 is arranged on the separator plate 2. Another sunk areas 44 of the upper cover plate 4 are configured to contain the electronic elements 53 which are arranged downwards on the lower surface of the circuit board 5.

In this embodiment, the circuit board 5 is positioned and mounted on the upper cover plate 4 and is provided with the adapter sealing member 52 corresponding to the sunk area 44 where the first sampling port 41, the second sampling port 42 and the third sampling port 43 are located, and the adapter sealing member 52 is provided with the first adapter portion 521, the second adapter portion 522 and the third adapter portion 523 corresponding to the first sampling port 41, the second sampling port 42 and the third sampling port 43.

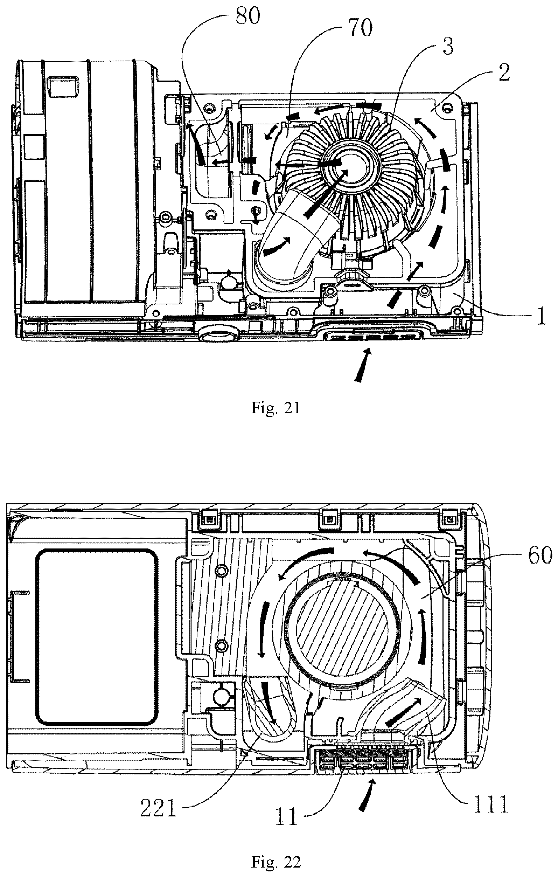

In this embodiment, the whole air flow direction may refer to Fig. 21, and a flow direction in each runner may refer to Fig. 22 to Fig. 25. The details are as follows.

The air intaking path is formed at the first cavity 60, a steering runner is formed at the steering pipe 220, the blowing runner is formed at the second cavity 70, and air entering through the air inlet 11 enters the first cavity 60, then passes through the air intaking path, the steering runner and the blowing runner to be blown into the flow monitoring cavity 80 by the air blower body 3 and then enters a humidifier 9 of the respirator.

The air inlet 11 communicates with the first cavity 60 through an air entering pipe 111 extending into the first cavity 60, and an internal opening of the air entering pipe 111 is located at a head end of an air flow path in the air intaking path.

The steering pipe 220 is provided with the first steering port 221 located in the first cavity 60, and a bottom opening of the first steering port 221 is located a tail end of the air flow path in the air intaking path and located at a head end of the air flow path in the steering runner.

The steering pipe 220 is provided with the second steering port 222 located in the second cavity 70, and a top opening of the second steering port 222 is located a tail end of the air flow path in the steering runner and located at a head end of the air flow path in the blowing runner.

The air entering port 31 of the air blower body 3 is located in the second cavity 70 and located at a tail end of the air flow path of the blowing runner. An air outlet port 32 of the air blower body 3 is located in the flow monitoring cavity 80.

This embodiment performs comprehensive optimized design on the respirator, especially for optimizing and improving a plurality of positions of the air path module of the respirator, to further achieve the objective of the present invention, which includes the following.
1. The first cavity 60 is composed of the housing 1 and the separator plate 2, and spare gaps in the original housing 1 may be utilized as the first cavity 60, so that air entering from the air inlet 11 can pass through the air intaking path, which may have the characteristics of optimizing an air path, making the air flow smoother, lowering noise, etc. in addition to reducing occupied space.
2. The whole structure of the steering pipe 220 is located on the separator plate 2 and the upper cover plate 4 and between the second cavity 70 and the first cavity 60, air entering the first cavity 60 from the air inlet 11 enters a first end of the steering pipe 220 along the air intaking path, then enters the second cavity 70 from a second end of the steering pipe 220 and enters the air blower body 3 via the blowing runner, and the steering pipe 220 is not arranged outside or on the periphery, so the external space of the air path module is not occupied, which also achieves space optimization.
3. The air flow monitoring region is arranged on the upper cover plate 4, the first sampling port 41 directly communicates with the first cavity 60, the second sampling port 42 directly communicates with the second cavity 70, the third sampling port 43 directly communicates with the flow monitoring cavity 80, the air flow in the air path module is monitored without dedicated arrangement of an extra air flow pipe, components such as pipes and joints are omitted, and the space is saved.
4. The plurality of air flow monitoring sensors on the circuit board 5 are jointly mated and connected with the first sampling port 41, the second sampling port 42 and the third sampling port 43 through the adapter sealing member 52, arrangement of the air flow monitoring region on the air path module is used ingeniously, when the circuit board 5 is arranged on an upper portion of the upper cover plate 4, the plurality of air flow monitoring sensors may be directly jointly matched and connected with the first sampling port 41, the second sampling port 42 and the third sampling port 43 through the adapter sealing member 52, some components are also omitted, fitting is simple, fitting efficiency and quality are improved, and the air flow monitoring sensors are also arranged towards the air path module, which also saves a part of space.

In particular, in a design of a detailed embodiment, it may be known from analysis of the above four points that embodiments of optimizing and improving functional designs are in close linkage and interdependent, so that the whole structure of the respirator is more compact and reliable, the number of components of the whole respirator is reduced, the structure is ingenious and reasonable, fitting is simple, fitting efficiency and fitting quality are improved, thus the safety and reliability of using the respirator by a patient are improved, and the respirator, as a medical supply, may make the patient use it more safely and more stably.

The above embodiments are only for explaining the technical concept and the features of the present invention and are intended to make those familiar with this technology understand and implement the contents of the present invention but not to limit the protection scope of the present invention. Equivalent variations or modifications made according to the spirit essence of the present invention are to be covered in the protection scope of the present invention.

## Claims

1. A respirator, comprising:
a housing (1), provided with an air inlet (11) communicating with outside atmosphere;
a separator plate (2), arranged on the housing (1) and provided with an air blower containing cavity (21), wherein an air blower body (3) is mounted in the air blower containing cavity (21); and
an upper cover plate (4), arranged on the separator plate (2);
wherein the respirator is configured that a first cavity (60) is formed between the separator plate (2) and the housing (1); a space between the separator plate (2) and the upper cover plate (4) is divided by the air blower body (3) into a second cavity (70) located in front of an air entering port (31) of the air blower body (3), and an air flow channel (22) communicating with the first cavity (60) and with the second cavity (70) is formed in the separator plate (2).

2. The respirator according to claim 1, wherein a flexible sealing member (23) is integrally formed by a mold, and located on partial surface and outer edge of the separator plate (2), the flexible sealing member (23) is configured to at least work on a connection position between the separator plate (2) and the housing (1) and on a connection position between the separator plate (2) and the upper cover plate (4).

3. The respirator according to claim 2, wherein a plurality of ribs (231) are extended out of the flexible sealing member (23) and arranged in the air blower containing cavity (21) of the separator plate (2) in a surrounding manner, and the plurality of ribs (231) constitute an elastic support for mounting the air blower body (3), so as to elastically limit the air blower body (3).

4. The respirator according to claim 2, wherein a first side plate (24) for separating the first cavity (60) is arranged at a lower side edge of the separator plate (2), a second side plate (12), corresponding to the first side plate (24) is arranged on the housing (1), a sealing strip (232) is extended out of the flexible sealing member (23) and arranged on an outer side of the first side plate (24); after the separator plate (2) is arranged in the housing (1), the first cavity (60) is separated by airtight cooperation of the first side plate (24), the sealing strip (232) and the second side plate (12); and the first side plate (24) and the second side plate (12) are configured to face inward and tilt downward.

5. The respirator according to claim 4, wherein an annular sealing ring (233) is further extended out of the sealing strip (232) of the flexible sealing member (23) and located on a round bottom surface of the separator plate (2), and the round bottom surface of the separator plate (2) is closely connected with the housing (1) through the sealing ring (233), so that an annular air intaking path is formed in the first cavity (60).

6. The respirator according to claim 1, wherein a space between the separator plate (2) and the upper cover plate (4) is divided by the air blower body (3) into a flow monitoring cavity (80) located behind an air outlet port (32) of the air blower body (3).

7. The respirator according to claim 6, wherein the upper cover plate (4) is provided with a first sampling port (41) communicating with the first cavity (60), a second sampling port (42) communicating with the second cavity (70) and a third sampling port (43) communicating with the flow monitoring cavity (80), and monitoring sensors (51) corresponding to the first sampling port (41), the second sampling port (42) and the third sampling port (43) are arranged on a circuit board (5) of the respirator.

8. The respirator according to claim 7, wherein the upper cover plate (4) is provided with a sunk area (44), and the first sampling port (41), the second sampling port (42) and the third sampling port (43) are arranged in the sunk area (44).

9. The respirator according to claim 8, wherein the circuit board (5) is provided with an adapter sealing member (52) corresponding to the sunk area (44) where the first sampling port (41), the second sampling port (42) and the third sampling port (43) are located.

10. The respirator according to any one of claims 7 to 9, wherein the first sampling port (41), the second sampling port (42) and the third sampling port (43) are joint pipe structures formed integrally with the upper cover plate (4), and the separator plate (2) is provided with a sampling extending pipe (25) configured to communicate with the first cavity (60) and with the first sampling port (41).

11. The respirator according to claim 8, wherein the upper cover plate (4) is provided with a plurality of sunk areas (44), and wherein another sunk area (44) is configured to accommodate an electronic element (53) which is arranged downwards on a lower surface of the circuit board (5).

12. The respirator according to claim 1, wherein a steering pipe (220) is arranged at the air flow channel (22) of the separator plate (2), the steering pipe (220) is provided with a first steering port (221) located in the second cavity (70) and a second steering port (222) located in the first cavity (60), and a laminar flow element (223) is arranged in the steering pipe (220).
